# EUROPEAN PATENT APPLICATION

(11) **EP 0 681 841 A1**
(43) Date of publication of application: **15.11.1995**
(21) Application number: 95901588.4
(22) Date of filing: 24.11.1994
(51) Int. Cl.: A61K 33/38, A61K 47/02

(54) **$i(IN VIVO) FREE-RADICAL GENERATOR**

(30) Priority: 26.11.1993 JP 321440/93
(71) Applicant: KIMURAKOGYO CO., LTD., Kanuma-shi, Tochigi-pref. (JP)
(72) Inventor: NOJIRI, Hisao, Tokyo 133 (JP); TAKASAKI, Yukei, Tochigi-pref. 321 (JP); TAKIZAWA, Fumio, Kimurakogyo Co., Ltd., Tochigi-pref. 322-05 (JP); OGIWARA, Masumiyo, Kimurakogyo Co., Ltd., Tochigi-pref. 322-05 (JP); KIMURA, Fumioki, Kimurakogyo Co., Ltd., Tochigi-pref. 322-05 (JP)
(74) Representative: Kraus, Walter, Dr.
(86) International application number: JP9401982
(87) International publication number: WO9514484

(57) **Abstract**

An *in vivo* free-radical generator comprising silver ions supported on a specified carrier. It generates free radicals .OH in the presence of water. When supported on an ion-exchanger, the silver ions release gradually .OH radicals in the presence of water. The released .OH radicals act on cancer cells to thereby suppress the cell growth and disrupt the cells, thus making cancer therapy possible. When zeolite or silica gel is used as the carrier in an amount below a specified value, the carrier is innocuous for the human body and can readily be excreted. Thus this generator is suitable as a medicine which is brought to the target tissue in the living body, such as a cancer cell, and generates free radicals thereat for long without adversely affecting the other tissues so much.

## Description

### TECHNICAL FIELD

This invention relates to an in vivo free-radical generator that generates free radicals within a living body such as the human body when brought to target tissues such as cancer cells or to the neighborhood thereof, which free radicals chemically act to break apart microbes, cells, tissues or the like that cause a disease, to thereby treat the disease.

### BACKGROUND ART

In general, chemically synthesized medicines prevails as medicines used to treat various diseases. There is also a field in which the medicinal efficacy of naturally occurring ingredients is utilized, as in herb medicines.

With regard to the chemically synthesized medicines, however, various problems of damage from chemicals are pointed out, including the problem of resistant cells produced by antibiotic substances and the problem of side effect of medicines.

For example, the treatment of cancer includes surgical treatment and medical treatment. In the medical approaches, radiations, laser beams, thermotherapy, embolectomy, chemotherapy and so forth are used. As chemotherapeutic agents, mitomycin, adriamycin, bleomycin, neocarzinostatin and so forth are used.

The treatment using these chemotherapeutic agents is, in principle, made by causing free radicals to cut DNA of cancer cells. More specifically, the chemotherapeutic agents produce active enzymes or free radicals acting against cancer cells, and break apart the cancer cells.

Stated more specifically, these chemotherapeutic agents respectively act in the following way.

### (1) Mitomycin, and adriamycin C:

These are quinone type anti-cancer agents, which generate semiquinone radicals by the action of NADH dehydrogenase existing in mitochondria, and the radicals transfer electrons to O₂ contained in the surrounding water to generate O₂⁻ (a superoxide) and ·OH (a hydroxyl radical). This ·OH breaks apart cancer cells.

### (2) Bleomycin:

This has no quinone structure. Hence, this agent itself does not turn into radicals as the mitomycin and adriamycin C do. That is, the O₂⁻ generated in a living organism reacts with metal ions, where it for the first time becomes possible to cut DNA. For example, in the presence of O₂⁻, it reacts with divalent ions of iron, so that ·OH is generated to break apart cancer cells.

### (3) Neocarzinostatin:

This protein combines with DNA to turn into radicals, to attack deoxyriboses of the nucleic acid, and also the presence of O₂⁻ causes generation of peroxy radicals (ROO·) to exhibit anti-cancer performance.

Without limitation to the treatment or prevention of cancer, methods of treating diseases by utilizing free radicals are presented, for example, in Motoharu Kondo, "What are Free Radicals?", K.K. Nippon Igakukan, pp.119-128.

Now, the chemotherapeutic agents as described above have hitherto had problems to be solved, as discussed below.

First, concerning the durability of medicinal efficacy, all the above chemotherapeutic agents generate ·OH or ROO· to break apart cancer cells. These generate ·OH or the like in a short time when brought into the body. They, however, have a very short duration for the medicinal efficacy.

Nevertheless, it is preferable for such free radicals to be generated little by little over a long period of time so that they act to enable complete cure. In practice, since it is impossible to be so, it is required to relatively frequently repeat the injection of chemotherapeutic agents that gives pain to patients.

In addition, while these chemotherapeutic agents generate free radicals to disrupt cancer tissues, they have the side effect of disrupting normal tissues also, or produce cells resistant to medicaments. Such problems have been pointed out.

The present invention was made taking note of the above points. An object thereof is to provide an in vivo free-radical generator that can generate free radicals for a relatively long time when brought into a living body and may cause no problems of side effect and production of resistant cells.

### DISCLOSURE OF THE INVENTION

The in vivo free-radical generator of the present invention, being brought to a target tissue in a living body, comprises silver ions supported on a carrier formed of an inorganic matter ion exchanger or an organic matter ion exchanger.

For example, this ion exchanger is formed of a porous material or a multilayered material.

In the present invention, an anti-fungus agent which has been already developed by the present inventors in order to prevent occurrence of fungi and is sprayed by means of a sprayer is used as a therapeutic agent. This is an agent comprising silver ions supported on a specific carrier. Experiments made by the present inventors have confirmed that silver ions supported on an ion exchanger cause radical decomposition of water to generate ·OH and ·H (a hydroradical). It has been also confirmed that new silver ions are supplied upon gradual release of silver ions from the carrier and hence the ·OH radicals are continuously generated. Also, a carrier as exemplified by zeolite or silica, when used in an amount not more than a given quantity, is harmless to the human body and can be readily excreted.

Thus, the in vivo free-radical generator of the present invention is suitable as a medicine for the therapy of cancer, which is brought to target tissues in a living body, such as cancer cells, and generates free radicals over a long period of time without causing side effect so much.

### BRIEF DESCRIPTION OF THE DRAWINGS

Fig. 1 illustrates in vivo free-radical generators according to the present invention.

Fig. 2 is a graph to show an ESR standard spectrum of hydroxyl radicals, for illustrating the action of the in vivo free-radical generator of the present invention.

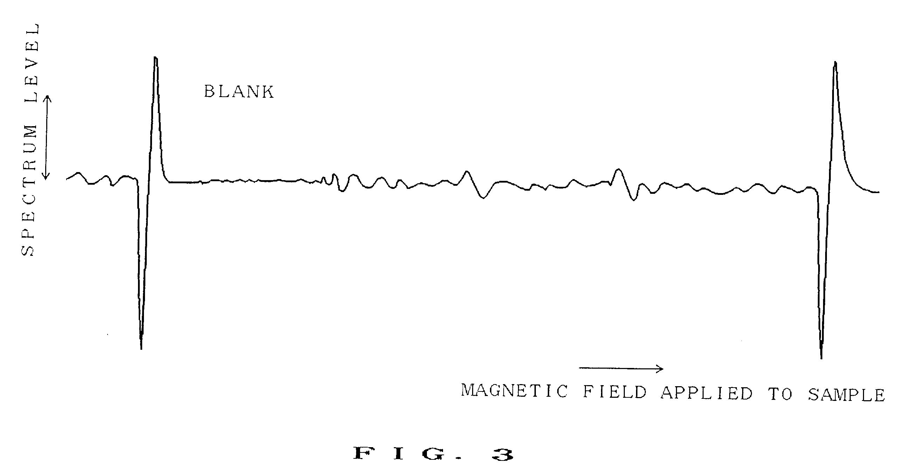

Fig. 3 is a graph to show an ESR spectrum in a blank test made using distilled water, for comparing it with the spectrum of the in vivo free-radical generator of the present invention.

Fig. 4 is a graph to show an ESR spectrum of the in vivo free-radical generator of the present invention in which zeolite is used as the carrier for silver ions.

Fig. 5 is a graph to show an ESR spectrum of the in vivo free-radical generator of the present invention in which silica gel is used as the carrier for silver ions.

### BEST MODE FOR WORKING THE INVENTION

The present invention will be described below in detail with reference to the examples shown in the drawings.

The in vivo free-radical generator of the present invention comprises silver ions supported on a specific carrier.

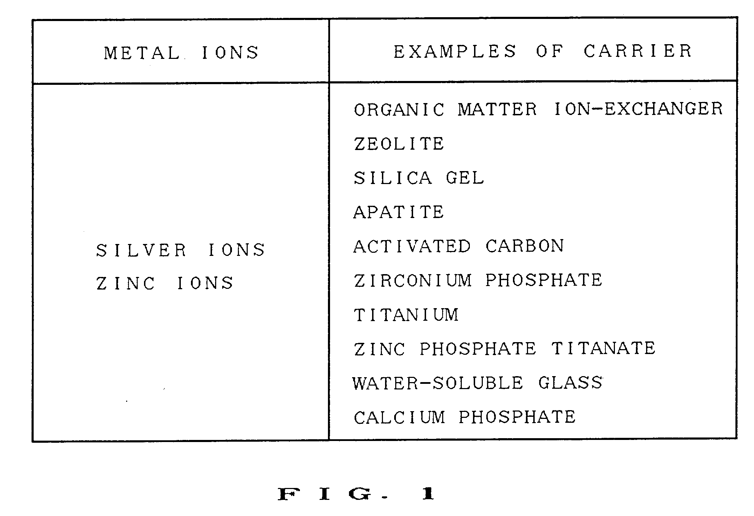

Fig. 1 illustrates the in vivo free-radical generator of the present invention.

As shown in Fig. 1, although there are various kinds of metal ions, silver ions are employed in the present invention. It is also preferable to incorporate a trace amount of zinc ions. As the carrier, any one of those shown in Fig. 1 or two or more of them in combination is/are suited. Stated specifically, an ion exchanger is preferred. As an inorganic matter ion exchanger, it includes zeolite, silica gel, apatite and zirconium phosphate.

It is also possible to use an organic matter ion exchanger. Of these carriers, those capable of adsorbing and holding silver are more preferable. For example, porous carriers such as silica gel and zeolite are very effective for maintaining the efficacy of medicines for a long term. A material with a high adsorptivity that enables adsorption of a large number of silver ions on its surface is also preferred as the carrier. Especially when medicines are required to have an immediate effect, a carrier that can support more silver ions on its surface is suited. As for the organic matter ion exchanger, any ion exchanger suited to the present object may be arbitrarily selected from those hitherto widely employed in ion-exchange membranes or the like.

These are also preferably used in the sate the ion exchanger is dissolved in a suitable solvent. The solvent may be distilled water, and as other solvents phosphates are suited. When silver ions are used in a higher concentration, solvents with many minus ions are preferred. Solvents capable of making silver ions kept hydrated for a long time are also preferred.

Heavy metals commonly have a antimicrobial action, and their antimicrobial power is considered to be stronger in the order of mercury, silver, copper, gold, zinc, iron and bismuth. However, in respect of heavy metal ions selected taking account of safety to the human body and antimicrobial action, it follows that silver ions are most suited among these. The safety of silver ions to the human body has been studied in the old Soviet Union over a period of 100 years or longer, and the results thereof are reported in L.A. Kriskie, "Silver-ionic Water", Kiev, 'Naucowadomca', 1987, translated by Keiichi Endo.

As reported therein, it has been ascertained that the one with a silver ion concentration of 200 ppm is harmful to the human body but is completely harmless when used in a concentration of 200 ppb to 500 ppb even when taken over a long period of time. According to the water quality standard in U.S.A. also, there is a judgement that drinking water with a silver ion concentration of 50 ppb is completely harmless even when taken over a period of 100 years.

In 1950, The U.S. Coast Guard also has given sanction to a lifesaving sea water desalting kit comprising silver ions supported on zeolite. This is a kit devised during World War II so that desalted water can be produced from sea water as drinking water for marine life boats, and intended for desalination and sterilization by the utilization of silver ions supported on zeolite. This proves the safety of silver ions to the human body. This kit is still put into use at present.

The free-radical generator of the present invention can also continue to release ·OH radicals in the body over a long period of time. Hence, it becomes possible to minimize the pain that may be given to patients when medicines are repeatedly injected to the patients.

In addition, the silver ions have no possibility of producing resistant cells and give no secondary damage from chemicals. Experiments made using radiation silver have also confirmed that silver ions concentrate in ulcer affected parts, where they hardly move therefrom. Hence, it becomes possible for them to continue to release ·OH radicals in the vicinity of cancer cells, so that, without their diffusion, a high therapeutic effect can be expected.

As for the carrier, silica gel is most preferred in view of the safety to the human body, the solubility of ions, the degree of dissociation and so forth. The silica gel is accepted as a food additive and has a high safety to the human body. Also, it is a porous material, has pore diameters of relatively as large as 70 to 100 angstroms, and is superior in the solubility of ions and the degree of dissociation. It still also has a high antimicrobial effect even in the presence of SH groups of proteins when incorporated into cells, and has a good suitability to medicines.

Moreover, the silica gel is water-soluble, having the advantage that it is readily excreted outside the body together with excreta after it has exhibited a given efficacy after administration, and can be said to be a material suited for decreasing side effects.

The silica gel is an ion exchanger and is capable of exchanging anions with silver ions of cyanol groups under weakly acidic conditions. In order to achieve a satisfactory antimicrobial action, silver may preferably be used in an amount of 3% in weight ratio with respect to the silica gel. In order to smoothen the gradual release of ·OH radicals, it is preferable to make zinc ions present together. Such zinc ions may be contained in an amount of, e.g., from about 2 to 3% in weight ratio.

For the purpose of administration to living bodies such as the human body, a silver-supported silica gel finely pulverized to have a particle diameter of about 1 micron is mixed in a suitable quantity of a solvent to form a slurry. This enables its administration to an affected part by the use of an injector or the like. As another method of administration, it may also be orally administered together with a solvent such as water.

In the case of, e.g., the treatment of leukaemia, a method may also be employed in which blood is taken out of the human body and, as in the same way as in what is called artificial dialysis, the cancer cells contained in the blood are brought into contact with the in vivo free-radical generator of the present invention, using an extracorporeal treating device, after which the blood thus treated is returned to the human body. In this instance, the free-radical generator can be effectively utilized when processed into antimicrobial particles as proposed by the present inventors (Japanese Patent Application No. 3-357635).

The above in vivo free-radical generator comprising silver ions supported on the silica gel acts to disrupt or break apart cancer cells by the mechanism as explained below.

First, once the in vivo free-radical generator is injected in the vicinity of cancer cells together with a solvent, the silver ions dissolve out in the water intracorporeally present inside and outside the cancer cells. The silver ions having thus dissolved out hydrate with the molecules of water.

The hydrated silver ions continuously decompose the molecules of water to generate hydroxyl radicals.

The reaction scheme of this mechanism is as shown below.
Scheme (1)

H₂O → ·H + ·OH

Here, the hydroxyl radicals break apart the cancer cells as previously described. Finally, the hydroxyl radicals are reduced to return to water. Hence, no harmful components can remain.

Normal cells neutralize ·OH by the action of intracellular SOD (superoxide dismutase) to make defense, but cancer cells have less SOD and are called defective cells having lost the defensive function. Because of such a characteristic of cancer cells, the cancer cells are known to have a lower resistance to ·OH than the normal cells. Accordingly, the treatment by such free radicals is very effective.

In addition, the cancer cells have a very higher proliferation potency than the normal cells, and hence have the nature to energetically incorporate their surroundings. Thus, it is presumed that, upon injection of the free-radical generator of the present invention in the vicinity of cancer cells, it is preferentially incorporated into the cancer cells and generates free radicals on the inside and outside of the cancer cells to break apart the cancer cells.

To prove the fact that the in vivo free-radical generator of the present invention actually generates the free radicals, Experiment 1 as reported below was made. The medicinal efficacy obtained when the free-radical generator of the present invention is actually used is proved by Experiment 2 subsequently shown below.

### Experiment 1

### - Establishment of Generation of Free Radicals -

To confirm the generation of free radicals, there are chemical methods and physical methods. The present inventors employed ESR spin trapping, which is a physical method. As devices for experiment, an ESR analyzer (REIX) and an aqueous solution cell (ES-LC20), manufactured by Nippon Denshi K.K., were used. Such a technique for confirming the generation of free radicals is disclosed in, e.g., "ESR and Free Radicals", edited by Hirotaka Nishikawa and Keiichi Yoshikawa, published by Nippon Igakukan.

Incidentally, among species of active oxygen, the compounds grouped into free radicals are highly reactive in aqueous solutions at room temperature and impose many difficulties in direct observation made using an analytical means.

To solve such a problem, a method proposed by E.G. Janzn is the spin trapping. This method is operated as described below.
i) Using a reagent (a spin trapping agent) capable of selectively reacting highly-reactive (short-lifetime) free radicals;
ii) the short-lifetime free radicals are supplemented (as one of reduction reaction), and then converted into longer-lifetime free radicals (called a spin adduct), in the state of which;
iii) their spectra are observed using a device (ESR) for selectively measuring free radicals only;
iv) then the information obtained is analyzed to qualitatively and quantitatively determine the free radicals.

Experiments to confirm the generation of free radicals were made according to the above steps.

As the standard spectrum for confirming (·OH), a spectrum of ·OH produced in the reaction of hydrogen peroxide water with a ferrous salt such as ferrous sulfate was used, which is widely known as Fenton's reaction.

The reaction scheme thereof is as follows:
Scheme (2)

H₂O₂ + Fe²⁺ = Fe³⁺ + ·OH

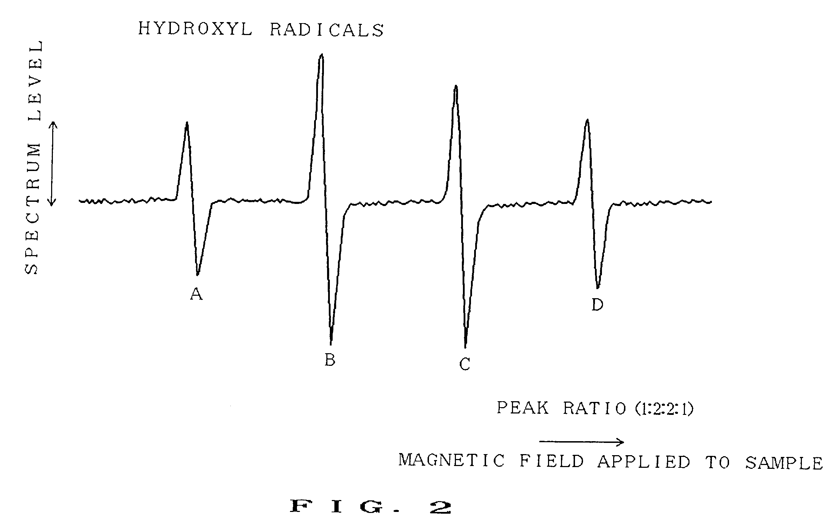

and the spectrum is as shown in Fig. 2. As shown therein, the absorption spectrum of hydroxyl radicals has four peaks in its wave form. In comparison of the heights of these peaks, they are in a ratio of 1:2:2:1. Hence, the detection of such a spectrum can prove the generation of ·OH radicals.

In the experiments, the following free-radical generators were used.
(1) A free-radical generator comprising 3% silver ions in weight ratio supported on zeolite (hereinafter called AgZ).
(2) A free-radical generator comprising 3% silver ions in weight ratio supported on silica gel (hereinafter called AgS).
   As the spin trapping agent, DMPO (5,5-dimethyl-pyrroline-1-oxide) was used.

Experiments were made to start with a blank test. To 180 microliters (µl) of ion-exchanged water, 20 µl of DMPO was added, which were thoroughly mixed, and then the mixture was put in the aqueous solution measuring cell to carry out analysis using the ESR analyzer.

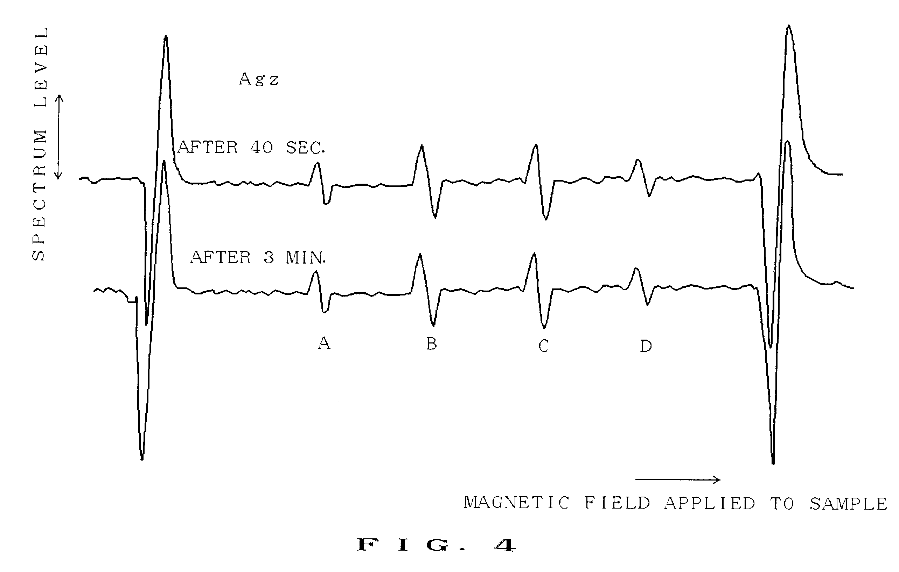

Results of this experiment are shown in Fig. 4. It shows the results of measurement on 40 seconds and 3 minutes after the mixing with DMPO, where the spectrum of ·OH is clearly seen. In comparison of the results of measurement on 40 seconds and 3 minutes after the mixing with DMPO, the spectrum has more grown after 3 minutes. Thus, it is possible to confirm the continuous generation and growth of radicals.

In the drawing, the graphs are drawn on reference axes shifted up and down so that the spectrum after 40 seconds can be compared with that after 3 minutes. The spectrum has grown by 1.25 times at "A" and by 1.1 times at "B".

An experiment was similarly made also on AgS. In 50 ml of high-purity ion-exchanged water, 50 mg of AgS was charged, and silver ion concentration in the resulting solution was measured. As the result, silver ions were in a concentration of 670 ppb.

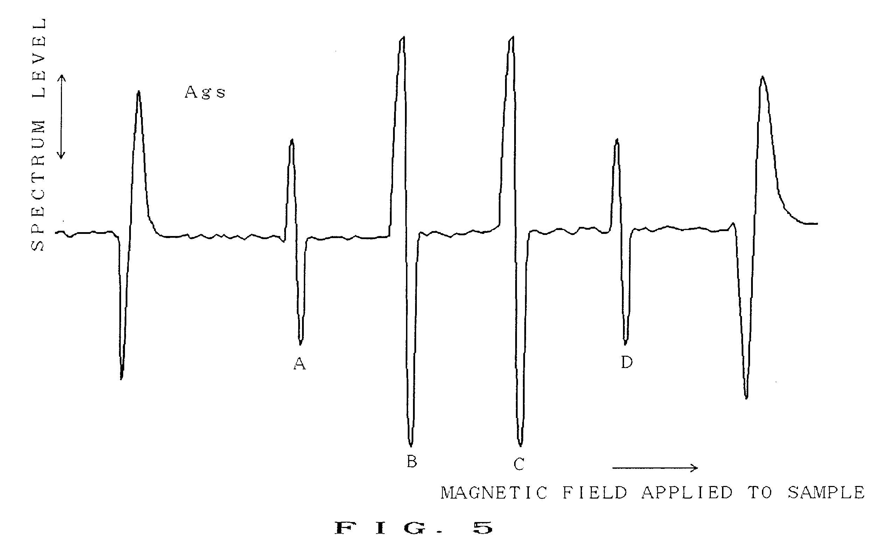

The reason why the silver ions are in a higher concentration than the case of AgZ is that the material used is highly dissociative into ions. Thus, conversely utilizing the difference in materials makes it possible to produce various kinds of free-radical generators according to purposes. Next, in the same way as previously done, 180 µl of this solution was taken out, and 20 µl of DMPO was added thereto, which were thoroughly mixed, and then the mixture was put in the aqueous solution measuring cell to carry out analysis using the ESR analyzer. Results obtained are shown in Fig. 5. The spectrum of ·OH is greater because of the higher ion concentration. The above experiments, for which the spectra measured on 40 seconds after the mixing with DMPO are graphically represented, have confirmed that the free-radical generator comprising silver ions supported on zeolite or silica gel continuously releases ·OH radicals in the presence of water molecules.

### Experiment 2

### - Establishment of Medicinal Efficacy -

In order to prove the medicinal efficacy of the free-radical generator of the present invention, the following experiment was made. First, as the generator the material comprising 3% silver ions in weight ratio supported on silica gel (AgS) was used.

The following cancer cells were used as test cells.
(1) Human acute promyelocytic leukemia cell strains HL-60
(2) Human esophageal squamous cancer cell strains ES-2
(3) Human malignant glioma cell strains A172
Next, as a culture solution for the cancer cells, 5% FCS-added DULBECCO'S MEM (DMEM) and 10% FCS-added RPMI 1640 were mixed in 1:1 and used for the human acute promyelocytic leukemia cell strains.

As culture solutions for the human esophageal squamous cancer cell strains and human malignant glioma cell strains, 5% FCC-added DMEM was used.

To the human acute promyelocytic leukemia cell strains cultured in the culture solution, the free-radical generator was added in an amount of 0.005% or 0.01% in weight ratio to the culture solution, and the strains were cultured for 24 hours in a CO₂ incubator to observe the results.

As a control, a silica gel on which no silver ions are supported was added in the same weight ratio to compare the results.

Results obtained were as shown in the following table.

| Kind of medicine/Amount | Survival rate of cancer cells in 0.005% addition | Survival rate of cancer cells in 0.01% addition |
|---|---|---|
| AgS | 62.6% | 56.4% |
| Control | 81.7% | 80.8% |

As is clear from the above results, the dependence of concentration on cancer cells and the biocidal effect have been confirmed.

Microscopic observation has also revealed that, as a feature of the disruption of cancer cells by the present free-radical generator, cell membranes were remarkably broken apart. Thus, a possibility of acting against membrane structures that are characteristic of cancer cells is expected.

Next, an experiment was made on the human malignant glioma cell strains.

To culture solutions containing the cancer cells, the free-radical generator was added at a rate ranging from 0.05% to 0.0005% in weight ratio to the culture solution, and the state of the cancer cells after 24 hours was observed. As a control, culture solutions prepared by adding, in the same weight ratios, silica gel to which no silver ions were added, were also observed. Results obtained were as shown in the following table.

| Rate of addition | Biocidal activities | Remarks |
|---|---|---|
| 0.05% | +++ | In 0.05% addition, the control shows a little poor growth. |
| 0.01% | ++ | |
| 0.005% | + | |
| 0.001% | ± | |
| 0.0005% | - | |

The reason why the control shows a little poor growth in 0.05% addition is presumed to be due to the culture conditions turned worse because of the silica gel layered on the cells.

As a whole, a biocidal effect clearly dependent on concentrations is seen in accordance with the amount of the in vivo free-radical generator added, and it is clear that such an effect is produced by the present free-radical generator.

Next, an experiment was made on the human esophageal squamous cancer cell strains. The experiment was made by adding to culture solutions 0.05% to 0.005% of the free-radical generator in weight ratio. Results obtained were as shown in the following table.

| Rate of addition | Biocidal activities | Remarks |
|---|---|---|
| 0.05% | + | More resistant to hydroxyl radicals than the human malignant glioma. |
| 0.01% | ± | |
| 0.005% | - | |

From the above experiments, it has been made clear that the free-radical generator is effective for the human cancer but with some differences in sensitivity depending on types.

### - Effects on Normal Cells -

However, a problem involved in medicines for treating cancer is, as stated also in the present specification, that they disrupt cancer cells and at the same time may damage or harm normal cells.

Studies were made on this point as reported below.

Effects on normal cells were examined in respect of human fetal lung fibroblast cells (IMR-90).

To make the experiment, the human fetal lung fibroblast cells were cultured in a culture flask, and the free-radical generator as used in the above experiment was added to make observation.

It was added in an amount of 0.05% in weight ratio to the culture solution, and how it progresses was observed. As a result, it was found that the normal cells were not broken apart at all even after 24 hours.

From such results, the present free-radical generator was confirmed not to disrupt normal cells so long as it is used in the therapy as described above.

Similar results were also obtained in respect of normal human umbilical intravenous endotherial cells.

Next, in order to ascertain general toxicity, a mutation test, an acute toxicity test and a dermal primary irritant test were made. The tests were requested to a juridical person Japan Food Analysis Center, and the results were reported on February 7 and 13, 1992 as Report No. OS-54120689-1 to 6.

According to the results, the free-radical generator was evaluated as follows:
(1) In the acute toxicity test (using mice), D₅₀ > 2,000 mg;
(2) in the mutation test (the Ministry of Labor, Notification No. 77), the mutagenesis is negative in the Ames test; and
(3) in the dermal primary irritant test (rabits), evaluated as weakly irritant matter according to the standard of Federal Register, Section 91, December, 1972.

In overall judgement from the foregoing results, the present free-radical generator can be evaluated as having a high safety to the human body.

The present invention is by no means limited to the above examples. As the carrier, any carriers may be used without limitation to organic types or inorganic types, so long as they are ion exchangers and their safety to the human body has been confirmed.

It has bee herein described that the in vivo free-radical generator according to the present invention continues to generate free radicals in a living body over a long period of time. The wording "over a long period of time" is specifically expressed in comparison with a certain time required for medicines to have an efficacy against cancer cells or the like to be treated. For example, assume that conventional medicines can exhibit an efficacy only for several hours, the in vivo free-radical generator of the present invention enables flexible adaptation to various types of treatment by adjustment to a period originally required, e,g, several hours, several days or several weeks, under suitable selection of carriers. Also, the silver ions made to adhere to the carrier surface can act to exhibit a therapeutic effect in a high immediate efficacy, and thereafter the silver ions incorporated inside the porous or multilayered carrier can act to generate free radicals for a subsequent certain period of time to increase the therapeutic effect or enhance the immunity. These can be adjusted by selecting the constitution and material for the above carrier or by selecting the particle diameter of the carrier, the solvent and so forth. Hence, the free-radical generator of the present invention can be well expected to effectively decrease the times of its administration compared with conventional therapy in which medicines are repeatedly administered.

The in vivo free-radical generator according to the present invention is also expected to have, in addition to the therapy of cancer, a wide range of medicinal efficacy as a therapeutic agent for various diseases described in the publications previously presented. For example, in experiments in foreign countries, made using silver-ionic water, clinical researches have been made on the following.

### (1) Surgery:

Disorder of bones, muscles, joints or the like caused by strepto- or staphylopneumococcus infectious bacillus and tubercle bucillus.

### (2) Ophthalomoloty:

Conjunctivitis, keratitis and other inflamations.

### (3) Otolaryngology:

Typanitis, pharyngitis, tonsillitis, rhinitis and mouthwashes.

### (4) Internal medicine:

Gastric ulcer, chronic gastritis, colitis and metabolism cacocymia.

### (5) Communicable diseases:

Dysentery, typhoid, paratyphoid, scarlatina and diphtheria.

### (6) Obsterics and Gynecology:

Inflamations of gynecological mucosa.

### (7) Dermatology:

Furuncle and dermatomycosis.

### (8) Stomatology:

Stomatitis, gingivitis and other stomatic diseases.

Thus, the free-radical generator of the present invention can provide environment in which silver ions are effectively injected into the body and also the silver ions stably generate free radicals. Hence, it can be expected that the present invention more expands the scope of therapeutic application in which the effect can be expected in conventional researches.

The in vivo free-radical generator of the present invention as described above comprises silver ions supported on an inorganic matter ion exchanger or an organic matter ion exchanger, and can continue to generate free radicals over a long period of time at an affected part when brought into the body, so that the therapeutic effect can be enhanced. Meanwhile, because of less harm in the material itself, it can be expected to have a superior medicinal efficacy in the treatment of cancer and other treatment, as a medicine having very little side effect.

Moreover, the free-radical generator of the present invention continuously effectively generate ·OH radicals effective for the treatment of cancer, in the presence of water. Hence, the same effect as the above can be obtained also when only the water in which silver ions have been dissolved out of the free-radical generator of the present invention is orally administered or directly applied to an affected part.

For this purpose, the product comprising silver ions supported on an inorganic matter ion exchanger or an organic matter ion exchanger may be mixed into a solvent such as water, and thereafter its supernatant may be obtained.

Since this supernatant itself has no ability to generate silver ions, it is preferable to, for example, orally administer it in portions several times a day.

## Claims

1. An in vivo free-radical generator, being brought to a target tissue in a living body, which comprises silver ions supported on a carrier formed of an inorganic matter ion exchanger or an organic matter ion exchanger.

2. The in vivo free-radical generator according to claim 1, wherein said ion exchanger is formed of a porous material or a multilayered material.

3. The in vivo free-radical generator according to claim 1 or 2, being used in the treatment of cancer, which comprises being brought to a part at which cancer cells are present.

4. A cancer therapeutic agent comprising a supernatant of a mixture obtained by dissolving in a solvent such as water a product comprising silver ions supported on an inorganic matter ion exchanger or an organic matter ion exchanger.
